# EUROPEAN PATENT APPLICATION

(11) **EP 4 390 828 A1**
(43) Date of publication of application: **26.06.2024**
(21) Application number: 22858147.6
(22) Date of filing: 15.06.2022
(51) Int. Cl.: G06Q 50/10

(54) **INFORMATION PROVISION SYSTEM**

(30) Priority: 16.08.2021 JP 2021132245
(71) Applicant: Cliniphar Ltd., Osaka-shi, Osaka, 540-0039 (JP)
(72) Inventor: TODA Kohei, Osaka-shi, Osaka 540-0039 (JP)
(74) Representative: Peters, Sebastian Martinus
(86) International application number: PCT/JP2022/023916
(87) International publication number: WO 2023/021823

(57) **Abstract**

[Problem] To provide an information provision system capable of meeting unmet needs and the like.

[Solution] An information provision system 1 that uses a computer having: a drug information storage means 3 for storing drug information that includes, for each drug, the name of the drug and information about countries in which the drug has been approved; an import drug information input means 5 for inputting import drug information that includes a drug name wanted to import and a country wanting the import; and an approval information output means 7 for reading, when import drug information related to a certain drug is input from the import drug input means 5, drug information related to the certain drug from the drug information storage means 3, and outputting approval information on whether the certain drug has been approved in a country which wants to import the certain drug.

## Description

### TECHNICAL FIELD

The present invention disclosure relates to an information provision system.

### BACKGROUND ART

Patent No. 3816326 describes a provision device for providing medicine related product information. The device utilizes an information communication terminal, which is connected to a network, to provide customers with medicine related products of merchandises and services that are useful for health care, including medicines.

Medicines are approved by some countries and not by others. For this reason, some medicines were only distributed in countries where they had been approved. Therefore, there was a problem that even when a medicine is effective, the medicine could not be distributed in a country where it had not been approved. On the other hand, there is a need to confirm the usefulness of some medicines even in a country where they have not been approved. In addition, even in a country where a medicine has not been approved, there may be another distributed medicine with the same active ingredient.

Patent Document 1: Japanese Patent No. 3816326

### SUMMARY OF INVENTION

### TECHNICAL PROBLEM

An object of the present invention is to provide a system that allows providing information necessary to provide opportunities for learning medicines and opportunities for procuring medicines.

### SOLUTION TO PROBLEM

An information provision system 1 disclosed in the present specification includes medicine information storage means 3, import medicine information input means 5, and approval information output means 7.

The medicine information storage means 3 is an element for storing medicine information including a name of a medicine and information on a country where the medicine is approved for each medicine.

The import medicine information input means 5 is an element for receiving import medicine information including a name of a medicine desired to be imported and a country to which the medicine is desired to be imported.

The approval information output means 7 is an element for reading out the medicine information on one medicine from the medicine information storage means 3 and outputting approval information as information on whether or not the one medicine is approved in a country to which the one medicine is desired to be imported when the import medicine information on the one medicine is input from the import medicine information input means 5.

A preferred embodiment of the approval information output means 7 is one that issues an alert when the one medicine is not approved in a country to which the one medicine is desired to be imported.

The medicine information may further include information on a target disease for each medicine.

It is preferred that the system 1 further includes target disease related medicine information output means 9. The target disease related medicine information output means 9 is an element for reading out information on the target disease related to the one medicine from the medicine information storage means 3 using the information on the target disease, reading out a name of a medicine related to the target disease from the medicine information storage means 3, and outputting the read name of the medicine as a medicine related to the one medicine.

Preferably, the medicine information further includes information on an active ingredient for each medicine.

It is preferred that the system 1 further includes same active ingredient medicine information output means 11.

The same active ingredient medicine information output means 11 is an element for reading out information on the active ingredient related to the one medicine from the medicine information storage means 3 using the information on the active ingredient, reading out a name of a medicine name including the active ingredient from the medicine information storage means 3, and outputting the read name of the medicine as a medicine having the same active ingredient as the one medicine.

The present specification also discloses a program for causing a computer to function as the above-described information provision system, and an information recording medium that can be read by a computer storing the program.

The present specification also discloses an information provision method using a computer. The information provision method includes storing medicine information including a name of a medicine and information on a country where the medicine is approved for each medicine; receiving import medicine information including a name of a medicine desired to be imported and a country to which the medicine is desired to be imported; and reading out the medicine information on one medicine and outputting approval information as information on whether or not the one medicine is approved in a country to which the one medicine is desired to be imported when the import medicine information on the one medicine is input.

### ADVANTAGEOUS EFFECTS OF INVENTION

The present disclosure allows providing the information provision system or the like that provide information necessary for providing opportunities for learning about the medicine and/or procuring the medicine. The system allows providing medicine information that meets unmet needs.

### BRIEF DESCRIPTION OF DRAWINGS

Fig. 1 is a block diagram illustrating an exemplary configuration of an information provision system.
Fig. 2 is a conceptual diagram illustrating an example of a system of the present invention.
Fig. 3 is a flowchart illustrating an operation example of the system.
Fig. 4 is a conceptual diagram illustrating an example of medicine information stored in a storage unit.

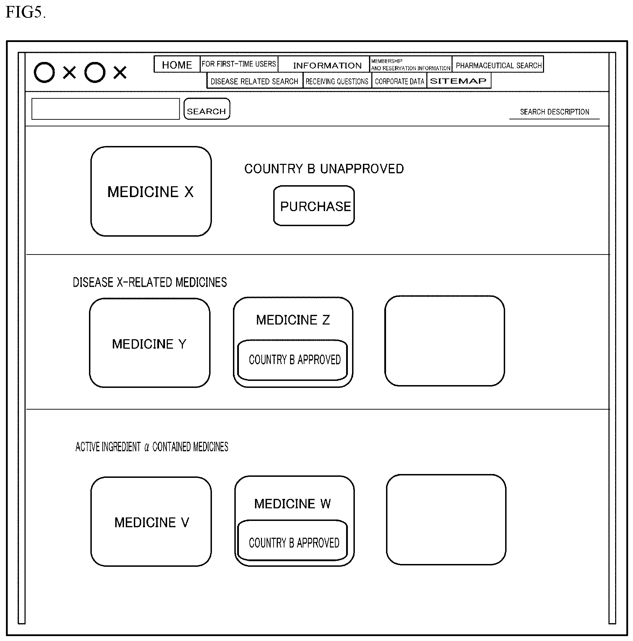

Fig. 5 is a conceptual diagram illustrating an output example of a terminal of a user.

### DESCRIPTION OF EMBODIMENTS

Hereinafter, embodiments of the present invention will be described with reference to the drawings. The present invention is not limited to the embodiments described below, and includes those modified as appropriate within the scope obvious to a person skilled in the art from the following embodiments.

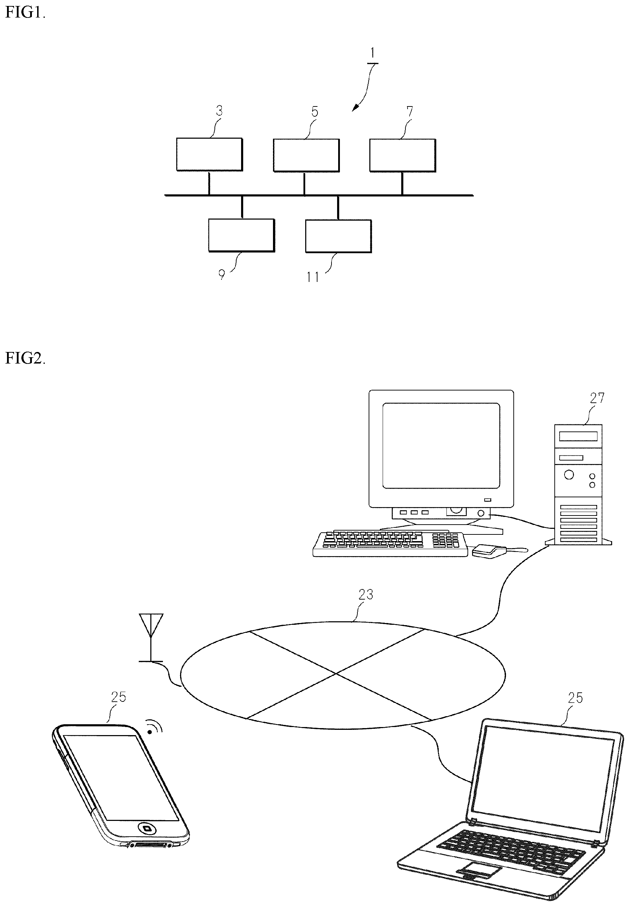

Fig. 1 is a block diagram illustrating an exemplary configuration of an information provision system. As illustrated in Fig. 1, the system 1 includes medicine information storage means 3, import medicine information input means 5, and approval information output means 7. As illustrated in Fig. 1, this system may further include one or both of target disease related medicine information output means 9 and/or same active ingredient medicine information output means 11. This system is a computer-based system.

Each means in the present specification can be achieved by each unit, each element, or each step in a computer. These may be achieved by software or hardware, or may be achieved by cooperation of software and hardware. The system may be implemented by a computer or a server-client system using computers and a server. The computer may be any or a combination of two or more of a mobile terminal, a desktop personal computer, and a server. These are usually connected so as to be able to exchange information via the Internet (intranet) or the like. A plurality of computers may be used to share functions, for example, some functions may be provided to one of the computers.

The computer includes an input unit, an output unit, a control unit, an arithmetic unit, and a storage unit, and each element is connected by a bus or the like so as to be able to exchange information. For example, a control program may be stored in the storage unit, or various kinds of information may be stored in the storage unit. When predetermined information is input from the input unit, the control unit reads out a control program stored in the storage unit. Then, the control unit reads out the information stored in the storage unit as appropriate, and transmits the information to the arithmetic unit. Further, the control unit transmits the appropriately input information to the arithmetic unit. The arithmetic unit performs arithmetic processes using the received various kinds of information, and stores them in the storage unit. The control unit reads out the calculation result stored in the storage unit and outputs the calculation result from the output unit. In this way, the various processes are executed. The various processes are executed by respective means and units.

Fig. 2 is a conceptual diagram illustrating an example of a system of the present invention. As illustrated in Fig. 2, the system 1 of the present invention (a system including the device of the present invention) may include a terminal 25 connected to the Internet or an intranet 23 and a server 27 connected to the Internet or the intranet 23. Of course, a single computer or a mobile terminal may function as a device of the present invention, or a plurality of servers may be present.

The medicine information storage means 3 is an element for storing the medicine information including the medicine name and information on the country where the medicine is approved for each medicine. The storage unit functions as the medicine information storage means 3.

Each medicine includes a commercially available medicine and a generic medicine thereof. The medicine name means the name of the medicine. An approved country means a country where each medicine is pharmaceutically approved. The medicine information including these are stored in the storage unit in association with, for example, ID (identification number) of the medicine. Each piece of information can then be used to read out information on each medicine. The medicine information may include information on the target disease and information on the active ingredient for each medicine.

The import medicine information input means 5 is an element for receiving the import medicine information including the name of the medicine desired to be imported and the country to which the medicine is desired to be imported. The input unit functions as the import medicine information input means 5. For example, a user inputs necessary information on a website using a keyboard and/or a mouse. The input information is then input into the system via the input unit of the system. In this way, the input unit of the system functions as the import medicine information input means 5.

The approval information output means 7 is an element for reading out the medicine information on the one medicine from the medicine information storage means 3 and outputting the approval information as information on whether or not the one medicine is approved in the country to which the one medicine is desired to be imported when the import medicine information on the one medicine is input from the import medicine information input means 5. Each element of the computer functions as the approval information output means 7. A preferred embodiment of the approval information output means 7 may issue an alert when the one medicine is not approved in the country to which the medicine is desired to be imported.

The target disease related medicine information output means 9 is an element for reading out information on a target disease related to the one medicine from the medicine information storage means 3 using the information on the target disease, reading out the medicine name related to the target disease from the medicine information storage means 3, and outputting the read name of the medicine as a medicine related to the one medicine. Each element of the computer functions as the target disease related medicine information output means.

The same active ingredient medicine information output means 11 reads out information on an active ingredient related to the one medicine from the medicine information storage means 3 using the information on the active ingredient, reads out the medicine name including the active ingredient from the medicine information storage means 3, and outputs the read name of the medicine as a medicine having the same active ingredient as the one medicine. Each element of the computer functions as the same active ingredient medicine information output means 11.

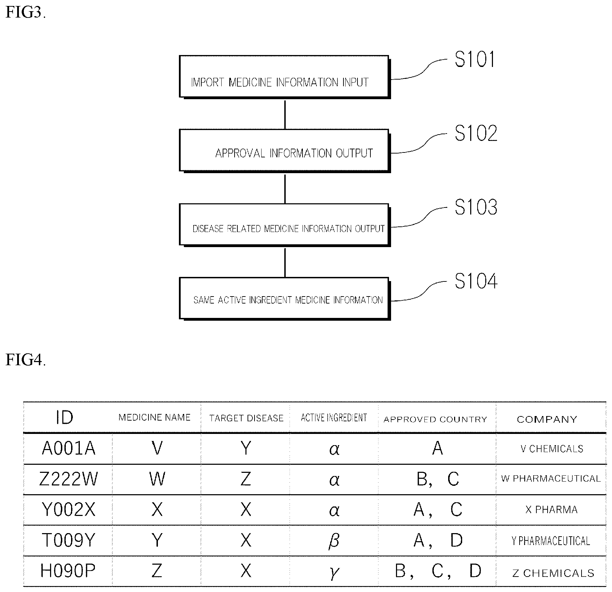

Next, an information provision method using this system will be described. Fig. 3 is a flowchart illustrating an operation example of this system. It should be noted that this flowchart is conceptual. For this reason, the order need not be as illustrated in this chart. Further, the present invention can be achieved by only one of the steps included in the flowchart. Any of the steps illustrated in this flowchart may be omitted. As a prerequisite, the storage unit of the system stores the medicine information. An example of the medicine information is as illustrated in Fig. 4. As illustrated in Fig. 4, the medicine information is preferably stored so as to be able to search for another item by using various items.

### (Import) Medicine Information Input Step (S101)

A user accesses a website. Information on a medicine name, a disease name, or an active ingredient is input to the website using a pointing device. The website may display various medicine names and allow selecting a medicine name by clicking the displayed medicine name. In this way, the medicine information input from a terminal of the user is input to the system via the Internet. The medicine information may be information on the medicine desired to be imported.

An address of the user or a mail destination of a product may be stored in the storage unit of the system. In this case, when the information on the user is input to the system, the system may read out country information stored in association with the address of the user or the mail destination of the product from the storage unit. In addition, the website may include a portion where a country to import the medicine is selected, and inputting the information from the terminal of the user in the portion allows inputting the information on the country to the system. In this way, the country information is input to the system. In this way, the import medicine information including the name of the medicine and the country to which the medicine is desired to be imported is input to the system.

### Approval Information Output Step (S102)

The control unit reads out the medicine information related to the medicine from the storage unit using the medicine information, such as the medicine name. The medicine information also includes information on the country in which the medicine is approved. The control unit causes the calculation unit to perform calculation of comparing the read out approved country with the input import-desired country. As a result, when the import-desired country is an approved country, a fact that the importing country is the approved country is outputted. Meanwhile, when the import-desired country is not an approved country, a fact that the importing country is not an approved country is outputted. At this time, in order to call attention, an alert may be given to a person who wishes to import. In this case, the control unit reads out information on the alert (display information or voice information) from the storage unit, and outputs the alert. Then, the alert is output from the terminal of the user. In any case, with the above-described steps, the approval information as information on whether or not the one medicine is approved in the country to which the one medicine is desired to be imported is output from the system, and output also from the terminal of the user.

This enables a user who attempts to import (purchase) one medicine to obtain information for determining whether or not the medicine is an approved medicine in the country to which the medicine is to be imported. Also, when the medicine is approved in the country, shipping the medicine from a branch of the country to be imported will allow the user to obtain the medicine inexpensively. In addition, when the medicine to be imported is not approved, this fact is displayed on the terminal of the user. Thus, the user can import the medicine after recognizing that the medicine is not approved in the country.

### Target Disease Related Medicine Information Output Step (S103)

In the previous step, the medicine information on the medicine was read out from the storage unit using the import medicine information, such as the medicine name. This medicine information may also include information on the target disease. The control unit reads out information on a disease related medicine, which is a medicine related to the same target disease, from the storage unit, using the information on the target disease. The system outputs the medicine information, which contains the name of the medicine for the same disease, as the disease related medicine. The terminal of the user receives the information on the disease related medicine and outputs the information to the output unit, such as a monitor. When the user inputs information on one medicine to the system in this way, other medicines that have the same target disease as the medicine are also displayed or the like. In this way, the information on the disease related medicine will also be displayed. The user possibly attempts to use this opportunity to purchase a relevant medicine, and the list information on the relevant medicines is also easily available. This step is particularly useful when the approval information output step is NO (when the import-desired country is not an approved country), and can provide information on medicine candidates other than the unapproved medicine for the target disease as appropriate.

### Same Active Ingredient Medicine Information Output Step (S104)

In the previous step, the medicine information on the medicine was read out from the storage unit using the medicine information, such as the medicine name. The medicine information may also include information on an active ingredient of the medicine. The control unit reads out information on the medicine having the same effect, which is a medicine including the same active ingredient, from the storage unit, using information on the active ingredient. Then, the control unit outputs the medicine information including the medicine name of the medicine having the same effect. The terminal of the user receives the medicine information including the medicine name of the medicine having the same effect, and outputs the medicine name of the medicine having the same effect to the output unit, such as a monitor.

Thus, when a user inputs information on one medicine to the system, information on other medicines that have the same active ingredient as the active ingredient of the medicine can be obtained. Then, even when the medicine is not approved in the country, the user can obtain information for purchasing a medicine having the same active ingredient that has been approved in the country.

This system is the information provision system. The information provision system causes a computer or a processor to store the medicine information including the name of the medicine and information on the approved country for each medicine, and to receive the import medicine information including the name of the medicine desired to be imported and the country to which the medicine is desired to be imported. When the aforementioned import medicine information on one medicine is input, the information provision system reads out the medicine information on the one medicine, and outputs the approval information as information on whether or not the one medicine is approved in the country to which the one medicine is desired to be imported.

The present specification also describes a program for causing a computer to function as the information provision system as described above. The present specification also provides an information recording medium that can be read by a computer storing such a program. Exemplary information recording media are CDs, CD-ROMs, DVDs, USB memories, and hard disks.

### EXAMPLES

For example, an example in which a server is present in Country A will be described. User B in Country B accesses Website A in Country A. Server A associated with Website A stores user information of User B. The user information of User B includes mailing information of the product of User B. The mailing information of the product of User B is the address in Country B. User B accesses Website A and designates Medicine X desired to be imported. At this time, User B may select Country B as the country to which Medicine X is to be imported. In addition, the system may read out the information of User B from the storage unit and select Country B as the importing country.

For example, information on Medicine X and Country B as an importing country is input into the system. At this time, the system reads out approved countries (Countries A and C) related to Medicine X from the storage unit. It should be noted that a target disease (Disease X) and an active ingredient (Ingredient α) of Medicine X may also be read out from the storage unit. The system then determines whether or not Country B is included in the read out approved countries. Then, the system outputs whether or not Medicine X is a medicine approved in Country B, which is the import-desired country, to a terminal of User B. The monitor (output unit) of the terminal of User B displays whether or not the medicine X is a medicine approved in Country B, which is the import-desired country.

Information on the target disease of Medicine X (Disease X) is read out from the storage unit. The system reads out the medicine names (Medicine Y, Medicine Z) and information on the approved countries (Medicine Y: Country A, Country D; Medicine Z: Country B, Country C, Country D) for other medicines stored in association with Disease X, from the storage unit. Then, the system outputs information on the read name of the medicines and the approved countries for the respective medicine names. The output unit of the terminal of the user displays the medicine information including the medicine names (Medicine Y, Medicine Z) other than Medicine X related to Disease X and the approved countries of the respective medicines (Medicine Y: Country A, Country D; Medicine Z: Country B, Country C, Country D).

Information on an active ingredient of Medicine X (Ingredient α) is read out from the storage unit. The system reads out the medicine names (Medicine V, Medicine W) and information on the approved countries (Medicine V: Country A; Medicine W: Country B, Country C) for other medicines stored in association with the active ingredient α, from the storage unit.

Then, the system outputs information on the approved countries related to the read name of the medicines and the respective medicine names. The output unit of the terminal of the user displays the medicine information including the medicine names (Medicine V, Medicine W) other than Medicine X with Ingredient α as an active ingredient and the information on the approved countries (Medicine V: Country A; Medicine W: Country B, Country C) for each medicine.

The above-described information is displayed on the terminal of User B. In consideration of the displayed information, User B can select a desired medicine and purchase an appropriate medicine using Website A. For a mechanism for purchasing a product and a mechanism for shipping a product, a mechanism of a known website may be used as appropriate.

### INDUSTRIAL APPLICABILITY

The system may be utilized at a commercial site or the like.

### REFERENCE SIGNS LIST

- 1: information provision system
- 3: medicine information storage means
- 5: import medicine information input means
- 7: approval information output means
- 9: target disease related medicine information output means
- 11: same active ingredient medicine information output means

## Claims

1. An information provision system (1) using a computer, comprising:
medicine information storage means (3) that stores medicine information including a name of a medicine and information on a country where the medicine is approved for each of medicines;
import medicine information input means (5) that receives import medicine information including a name of a medicine desired to be imported and a country to which the medicine is desired to be imported; and
approval information output means (7) that reads out the medicine information on one medicine from the medicine information storage means (3) and outputs approval information as information on whether or not the one medicine is approved in a country to which the one medicine is desired to be imported when the import medicine information on the one medicine is input from the import medicine information input means (5).

2. The information provision system (1) according to claim 1, wherein
the approval information output means (7) issues an alert when the one medicine is not approved in a country to which the one medicine is desired to be imported.

3. The information provision system (1) according to claim 1, wherein
the medicine information further includes information on a target disease for each of the medicines, and
the information provision system (1) further comprises target disease related medicine information output means (9) that reads out information on the target disease related to the one medicine from the medicine information storage means (3) using the information on the target disease, reads out a name of a medicine related to the target disease from the medicine information storage means (3), and outputs the read name of the medicine as a medicine related to the one medicine.

4. The information provision system (1) according to claim 1, wherein
the medicine information further includes information on an active ingredient for each of the medicines, and
the information provision system (1) further comprises same active ingredient medicine information output means (11) that reads out information on the active ingredient related to the one medicine from the medicine information storage means (3) using the information on the active ingredient, reads out a name of a medicine including the active ingredient from the medicine information storage means (3), and outputs the read name of the medicine as a medicine having the same active ingredient as the one medicine.

5. A program for causing a computer to function as an information provision system using the computer, the program causing the information provision system to function as:
medicine information storage means (3) that stores medicine information including a name of a medicine and information on a country where the medicine is approved for each medicine;
import medicine information input means (5) that receives import medicine information including a name of a medicine desired to be imported and a country to which the medicine is desired to be imported; and
approval information output means (7) that reads out the medicine information on one medicine from the medicine information storage means (3) and outputs approval information as information on whether or not the one medicine is approved in a country to which the one medicine is desired to be imported when the import medicine information on the one medicine is input from the import medicine information input means (5).

6. An information recording medium readable by a computer storing the program according to claim 5.

7. An information provision method using a computer, comprising:
storing medicine information including a name of a medicine and information on a country where the medicine is approved for each of medicines;
receiving import medicine information including a name of a medicine desired to be imported and a country to which the medicine is desired to be imported; and
reading out the medicine information on one medicine and outputting approval information as information on whether or not the one medicine is approved in a country to which the one medicine is desired to be imported when the import medicine information on the one medicine is input.
